# EUROPÄISCHE PATENTANMELDUNG

(11) **EP 3 318 994 A1**
(43) Veröffentlichungstag der Anmeldung: **09.05.2018**
(21) Anmeldenummer: 16020437.6
(22) Anmeldetag: 07.11.2016
(51) Int. Cl.: G06F 19/00

(54) **SYSTEM ZUR FORTSCHREIBUNG EINER ELEKTRONISCHEN PATIENTENAKTE**

(71) Anmelder: König, Silke, 31789 Hameln (DE)
(72) Erfinder: König, Silke, 31789 Hameln (DE)
(74) Vertreter: Kayser, Christoph

(57) **Zusammenfassung**

Ein System zur Fortschreibung einer elektronischen Patientenakte mit einem Haupt-Netzwerk, einer in dem Haupt-Netzwerk integrierten Datenbank, einer Mehrzahl von Mobilgeräten, die eine Zugangsberechtigung zu dem Haupt-Netzwerk haben und mit einer auf den Mobilgeräten installierten Anwendungs-App, ist dadurch gekennzeichnet, dass ein Benutzer des Mobilgerätes über die Anwendungs-App wenigstens einen gesundheitsbezogenen Patientendatensatz eines Patienten A eingeben kann, der dann über eine mobile Datenverbindung in der integrierten Datenbank hinterlegten elektronischen Patientenakte gespeichert wird, wobei der in der Patientenakte des Patienten A aktuell gespeicherte Patientendatensatz ein Alarmsignal bei einem in das Haupt-Netzwerk eingebundenen medizinischen Fachpersonal auslöst, wenn der gespeicherte Patientendatensatz allein oder in Kombination mit bereits vorher in derselben Patientenakte des Patienten A gespeicherten Patientendatensätzen einen vorbestimmten kritischen Wert erreicht.

## Beschreibung

Die vorliegende Erfindung betrifft ein System zur Fortschreibung einer elektronischen Patientenakte mit einem Haupt-Netzwerk, einer in dem Haupt-Netzwerk integrierten Datenbank, einer Mehrzahl von Mobilgeräten, die eine Zugangsberechtigung zu dem Haupt-Netzwerk haben und mit einer auf den Mobilgeräten installierten Anwendungs-App.

Ein solches System ist aus dem allgemeinen Stand der Technik bekannt. Es gibt heute eine Vielzahl von Anwendung-Apps, mit deren Hilfe über ein drahtloses Netzwerk Zugriff auf eine Datenbank erlangt werden kann, auch dann, wenn der Zugriff eine Zugangsberechtigung erfordert. Es ist auch bekannt, Inhalte eines Mobilgerätes über ein Netzwerk mit Inhalten einer Datenbank zu synchronisieren. Die bisher bekannten Systeme sind aber nicht dazu geeignet, für die Fortschreibung einer elektronischen Patientenakte genutzt zu werden.

Zum Beispiel ist es die Führung einer elektronischen Patientenakte unerlässlich, dass die über ein Mobilgerät eingegebenen digitalen Patientendaten unmittelbar an eine Datenbank übertragen werden, um diese aktuell zu halten. Es geht also nicht, solche neu eingegebenen digitalen Patientendaten zunächst auf dem Mobilgerät zu speichern und dann zu einem späteren Zeitpunkt eine Synchronisierung der Datenbank durchzuführen. Bei aktuellen Systemen dient das Mobilgerät im Wesentlichen dazu, Informationen aus der elektronischen Patientenakte abzufragen. Es ist also für das medizinische Personal möglich, in der Nähe eines Patienten über ein Mobilgerät in der elektronischen Patientenakte zu lesen. Es ist zudem auch möglich, dass medizinisches Personal über ein Mobilgerät in die elektronische Patientenakte neue Einträge schreibt. Ein Nachteil ist aber, dass ein Ergebnis der Fortschreibung ausbleibt. So können unterschiedliche Personen zu unterschiedlichen medizinischen Sachverhalten Einträge in die elektronische Patientenakte vornehmen, ohne dass die elektronische Patientenakte oder irgendjemand anderes reagiert. In der Regel wird immer nur das lesende medizinische Personal Rückschlüsse aus der elektronischen Patientenakte ziehen können. Die elektronische Patientenakte wird also immer erst dann geöffnet und angeschaut, wenn diese gelesen werden soll.

Die Aufgabe der vorliegenden Erfindung ist, ein System zur Fortschreibung einer elektronischen Patientenakte derart weiterzubilden, dass das medizinische Personal auf eine kritische Änderung in der elektronischen Patientenakte aufmerksam gemacht wird.

Die Aufgabe wird erfindungsgemäß dadurch gelöst, dass ein Benutzer des Mobilgerätes über die Anwendungs-App wenigstens einen gesundheitsbezogenen Patientendatensatz eines Patienten A eingeben kann, der dann über eine mobile Datenverbindung in der integrierten Datenbank hinterlegten elektronischen Patientenakte gespeichert wird, wobei der in der Patientenakte des Patienten A aktuell gespeicherte Patientendatensatz ein Alarmsignal bei einem in das Haupt-Netzwerk eingebundenen medizinischen Fachpersonal auslöst, wenn der gespeicherte Patientendatensatz allein oder in Kombination mit bereits vorher in derselben Patientenakte des Patienten A gespeicherten Patientendatensätzen einen vorbestimmten kritischen digitalen Wert erreicht.

Mit dem erfindungsgemäßen System ist es möglich, dass zum Beispiel Ärzte unterschiedlicher Fachrichtungen und auch Patienten ihren gesundheitlichen Zustand über das Mobilgerät dokumentieren und diese Dokumentation in der elektronischen Patientenakte speichern. In der elektronischen Patientenakte werden die zu den Befunden des medizinischen Personals und zu den Dokumentationen der Patienten Bewertungen durchgeführt, die im Ergebnis einen digitalen Wert erzeugen. Solange der digitale Wert unter einem vorbestimmten kritischen Wert liegt, äußert sich die elektronische Patientenakte nicht. Erst wenn der gespeicherte Patientendatensatz allein oder in Kombination mit bereits vorher in derselben Patientenakte gespeicherten Patienten Datensätzen, also die Kombination aus Befunden und Dokumentationen aller Beteiligten, den vorbestimmten kritischen digitalen Wert erreicht, löst die elektronische Patientenakte ein Alarmsignal aus, welches dem medizinischen Fachpersonal signalisiert, dass der gesundheitliche Zustand des betreffenden Patienten medizinisch überprüft werden muss.

In einem solchen Fall wird sich zum Beispiel der in das System eingebundene Hausarzt bei dem betreffenden Patienten A melden und ihn zu einer Gesundheitskontrolle in seine Praxis bitten oder einen Hausbesuch gleichem Zweck ankündigen.

Ein weiterer Vorteil der vorliegenden Erfindung ist, dass wenigstens ein Mobilgerät wenigstens einen Teil der Patientenakte herunterladen kann. Dadurch wird ermöglicht, dass zum Beispiel der in das System eingebundene Hausarzt auch bei einem Hausbesuch Zugriff auf sämtliche gesundheitsbezogenen Patientendaten, also auch Patientendaten, die durch andere Fachärzte in die elektronische Patientenakte eingeschrieben worden sind, zuzugreifen und ohne großen Datentransfer offline verwenden kann.

Ein weiterer Vorteil der vorliegenden Erfindung ist, dass zusätzlich eine Mehrzahl von Teil-Netzwerken an das Haupt-Netzwerk angebunden sind, wobei jedes Teil-Netzwerk Patientendatensätze des Patienten bereitstellen kann, die über eine Datenübertragungseinrichtung in der elektronischen Patientenakte des Patienten A gespeichert werden können. Durch die Anbindung mehrerer Teil-Netzwerke an das Haupt-Netzwerk können gesundheitsbezogene Patienten Datensätze aus unterschiedlichen Netzwerken in der im zentralen Haupt-Netzwerk eingebunden den Datenbank gespeichert werden. Auf diese Weise wird sichergestellt, dass wirklich alle Patientendaten in einer einzigen elektronischen Patientenakte zusammengeführt werden können.

Eine Ausführungsform der vorliegenden Erfindung wird im Folgenden anhand der Zeichnungen näher beschrieben. Es zeigen:
Figur 1 eine grafische Darstellung des Systems gemäß vorliegender Erfindung mit einem Haupt-Netzwerk und daran angebunden Teil-Netzwerken; und
Figur 2 eine schematische Darstellung eines Screen-Designs einer App gemäß vorliegender Erfindung.

Die vorliegende Erfindung bezieht sich auf ein digitales Umfeld. In der nachfolgenden Beschreibung zu der Erfindung werden die verwendeten Begriffe in folgendem Sinne verwendet:

### Elektronische Patientenakte

Unter einer elektronischen Patientenakte ist im Kontext der vorliegenden Erfindung ein digitaler Speicherort in einer Datenbank gemeint.

### Netzwerk

Mit einem Netzwerk ist im Kontext der vorliegenden Erfindung ein Netzwerk elektronischer Geräte gemeint.

### Datenbank

Mit einer Datenbank ist im Kontext der vorliegenden Erfindung eine elektronische Einrichtung zur Speicherung digitaler Daten gemeint.

### Anwendungs-App

Mit einer Anwendungs-App ist im Kontext der vorliegenden Erfindung eine Anwendungssoftware oder Applikation gemeint, die genutzt wird, um eine nützliche oder gewünschte nicht systemtechnische Funktionalität zu bearbeiten oder zu unterstützen.

### Gesundheitsbezogener Patientendatensatz

Mit einem gesundheitsbezogenen Patientendatensatz ist im Kontext der vorliegenden Erfindung ein Satz digitaler Daten gemeint, der einem Gesundheitszustand eines Patienten entspricht.

### Mobile Datenverbindung

Mit einer mobilen Datenverbindung ist im Kontext der vorliegenden Erfindung jede Zugangstechnologie 2G, 3G, 4G, 5G, LTE etc. und auch WLAN oder Satellitenübertragung gemeint. Auch zukünftige Übertragungstechniken sollen mit eingeschlossen sein, da die Übertragungstechnik nicht Gegenstand der vorliegenden Erfindung ist.

### Mobilgerät

Mit Mobilgerät ist ein mobiles Endgerät gemeint, dass aufgrund seiner Größe und seines Gewichts ohne größere körperliche Anstrengung tragbar und somit mobil einsetzbar ist und als ein Endgerät zu verstehen ist, das informationstechnisch und kommunikationstechnisch zum Empfang und zur Übersendung digitaler Daten geeignet ist.

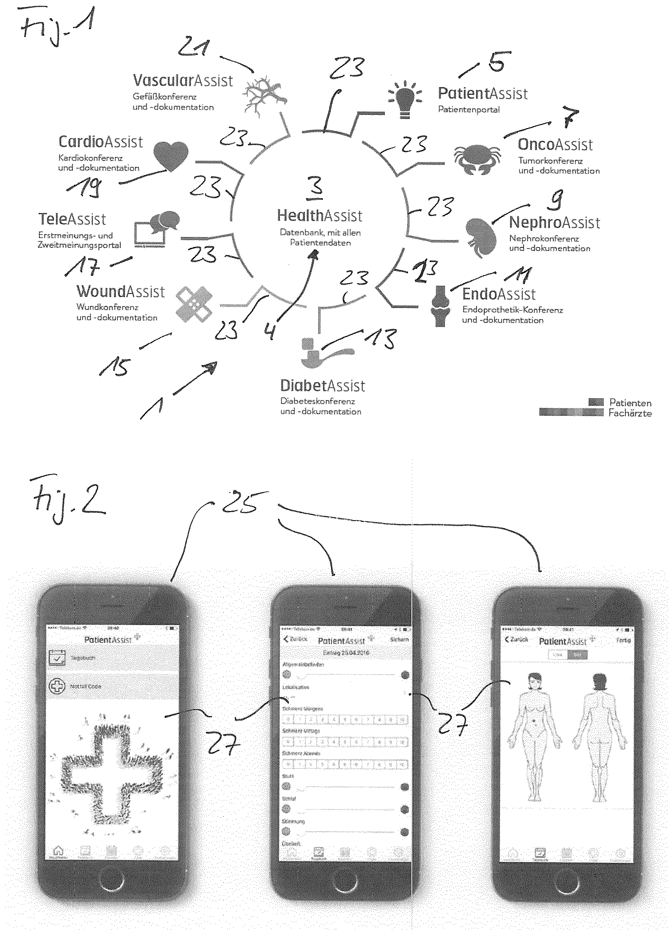

In Figur 1 ist schematisch dargestellt, wie das System 1 gemäß vorliegender Erfindung aufgebaut ist. Ein zentrales Haupt-Netzwerk 3 umfasst eine Datenbank 4, in der eine elektronische Patientenakte mit Patientendaten in digitaler Form angelegt ist. An das zentrale Haupt-Netzwerk 3 sind eine Mehrzahl von Teil-Netzwerken 5, 7, 9, 11, 13, 15, 17, 19, 21, angebunden. Das Teil-Netzwerk 5 ist ein Patientenportal, über das Patienten ihren Gesundheitszustand dokumentieren können und digitale Daten und Datensätze erzeugen können, die über eine mobile Datenverbindung 23 in der einem jeweiligen Patienten A zugeordneten Patientenakte in der Datenbank 4 gespeichert werden können.

Das Teil-Netzwerk 7 ist zum Beispiel einer Tumorkonferenz und-dokumentation zugeordnet. Auch in dem Teil-Netzwerk 7 werden digitale Daten und Datensätze erzeugt, die den Gesundheitszustand zum Beispiel des Patienten A betreffen und über die mobile Datenverbindung 23 in der Patientenakte des Patienten A gespeichert werden können.

Das Teil-Netzwerk 9 ist zum Beispiel einer Nephrologiekonferenz und -dokumentation zugeordnet. Auch in dem Teil-Netzwerk 9 werden digitale Daten und Datensätze erzeugt, die der Gesundheitszustand zum Beispiel des Patienten A betreffen und über die mobile Datenverbindung 23 in der Patientenakte des Patienten A gespeichert werden können.

Das Teil-Netzwerk 11 ist zum Beispiel einer Endoprothetik-Konferenz und -dokumentation zugeordnet. Auch in dem Teil-Netzwerk 11 werden digitale Daten und Datensätze erzeugt, den Gesundheitszustand zum Beispiel des Patienten A betreffen und über die mobile Datenverbindung 23 in der Patientenakte des Patienten A gespeichert werden können.

Das Teil-Netzwerk 13 ist zum Beispiel einer Diabeteskonferenz und -dokumentation zugeordnet. Auch in dem Teil-Netzwerk 13 werden digitale Daten und Datensätze erzeugt, die den Gesundheitszustand zum Beispiel des Patienten A betreffen und über die mobile Datenverbindung 23 in der Patientenakte des Patienten A gespeichert werden können.

Das Teil-Netzwerk 15 ist zum Beispiel einer Wundkonferenz und -dokumentation zugeordnet. Auch in dem Teil-Netzwerk 15 werden digitale Daten und Datensätze erzeugt, die den Gesundheitszustand zum Beispiel des Patienten A betreffen und über die mobile Datenverbindung 23 in der Patientenakte des Patienten A gespeichert werden können.

Das Teil-Netzwerk 17 ist zum Beispiel einem Erstmeinungs -und Zweitmeinungsportal zugeordnet. Auch in dem Teil-Netzwerk 17 werden digitale Daten und Datensätze erzeugt, die den Gesundheitszustand zum Beispiel des Patienten A betreffen und über die mobile Datenverbindung 23 in der Patientenakte des Patienten A gespeichert werden können.

Das Teil-Netzwerk 19 ist zum Beispiel einer Kardiokonferenz und -dokumentation zugeordnet. Auch in dem Teil-Netzwerk 19 werden digitale Daten und Datensätze erzeugt, die den Gesundheitszustand zum Beispiel des Patienten A betreffen und über die mobile Datenverbindung 23 in der Patientenakte des Patienten A gespeichert werden können.

Das Teil-Netzwerk 21 ist zum Beispiel einer Gefäßkonferenz und -dokumentation zugeordnet. Auch in dem Teil-Netzwerk 21 werden digitale Daten und Datensätze erzeugt, die den Gesundheitszustand zum Beispiel des Patienten A betreffen und über die mobile Datenverbindung 23 in der Patientenakte des Patienten A gespeichert werden können.

In Figur 2 ist schematisch ein Screendesign der Anwendungs-App 27 gemäß vorliegender Erfindung dargestellt, die auf einem Mobilgerät 25 eines Patienten, zum Beispiel des Patienten A, gespeichert ist. Nach Aufruf der App öffnet sich dem Patienten A das in Figur 2 dargestellte Screendesign. Der Patient A kann über diese App zum Beispiel ein Tagebuch führen oder einen Notfall eingeben. Für den Eintrag in ein Tagebuch wird dem Patienten A eine vereinfachte Eingabemöglichkeit angezeigt. So wird von ihm nicht verlangt, beliebige Texte einzugeben, sondern stattdessen kann er den Grad seines Allgemeinbefinden, seiner Schmerzen morgens, mittags und/oder abends sowie seinen Eindruck über seinen Stuhlgang, seinen Schlaf, seine Stimmung, seiner Übelkeit oder dergleichen mithilfe eines Schiebers oder eines Zahlenwertes zwischen 1 und 10 festlegen. Zudem wird ihm mit der App auch eine Ansicht des menschlichen Körpers Vorderansicht und Rückansicht dargestellt, sodass er den Ort eines Schmerzes, eines Unbehagens oder dergleichen durch Berührung festlegen kann. Diese Möglichkeiten werden hier nur beispielhaft aufgezählt und sind keinesfalls als beschränkend anzusehen.

### Bezugszeichenliste

- 1: System
- 3: Haupt-Netzwerk
- 4: Datenbank
- 5: Teil-Netzwerk
- 7: Teil-Netzwerk
- 9: Teil-Netzwerk
- 11: Teil-Netzwerk
- 13: Teil-Netzwerk
- 15: Teil-Netzwerk
- 17: Teil-Netzwerk
- 21: Teil-Netzwerk
- 23: mobile Datenverbindung
- 25: Mobilgerät
- 27: Anwendungs-App

## Patentansprüche

1. System (1) zur Fortschreibung einer elektronischen Patientenakte mit einem Haupt-Netzwerk (3), einer in dem Haupt-Netzwerk (3) integrierten Datenbank(4), einer Mehrzahl von Mobilgeräten (25), die eine Zugangsberechtigung zu dem Haupt-Netzwerk (3) haben und mit einer auf den Mobilgeräten (25) installierten Anwendungs-App (27),
**dadurch gekennzeichnet,**
**dass** ein Benutzer des Mobilgerätes (25) über die Anwendungs-App (27) wenigstens einen gesundheitsbezogenen Patientendatensatz eines Patienten A eingeben kann, der dann über eine mobile Datenverbindung (23) in der integrierten Datenbank (4) hinterlegten elektronischen Patientenakte gespeichert wird, wobei der in der Patientenakte des Patienten A aktuell gespeicherte Patientendatensatz ein Alarmsignal bei einem in das Haupt-Netzwerk (3) eingebundenen medizinischen Fachpersonal auslöst, wenn der gespeicherte Patientendatensatz allein oder in Kombination mit bereits vorher in derselben Patientenakte des Patienten A gespeicherten Patientendatensätzen einen vorbestimmten kritischen Wert erreicht.

2. System nach Anspruch 2,
**dadurch gekennzeichnet,**
**dass** wenigstens ein Mobilgerät (25) wenigstens einen Teil der Patientenakte herunterladen kann.

3. System nach einem der Ansprüche 1 oder 2,
**dadurch gekennzeichnet,**
**dass** zusätzlich eine Mehrzahl von Teil-Netzwerken (5; 7; 9; 11; 13; 15; 17; 21) an das Haupt-Netzwerk (3) angebunden sind, wobei jedes Teil-Netzwerk (5; 7; 9; 11; 13; 15; 17; 21) Patientendatensätze des Patienten bereitstellen kann, die über die mobile Datenverbindung (23) in der elektronischen Patientenakte des Patienten A gespeichert werden können.

4. System nach Anspruch 3,
**dadurch gekennzeichnet,**
**dass** wenigstens ein Teilnehmer eines Teil-Netzwerks (5; 7; 9; 11; 13; 15; 17; 21) wenigstens einen Teil der elektronischen Patientenakte herunterladen kann.
